# EUROPEAN PATENT APPLICATION

(11) **EP 4 190 887 A1**
(43) Date of publication of application: **07.06.2023**
(21) Application number: 20947049.1
(22) Date of filing: 29.07.2020
(51) Int. Cl.: C12M 1/12, C02F 1/28, B01D 39/18

(54) **NANOCELLULOSE SUPPORT AND METHOD FOR PRODUCING SAME**

(71) Applicant: LG Electronics Inc., Yeongdeungpo-Gu Seoul 07336 (KR)
(72) Inventor: PARK, Koun, Seoul 06772 (KR); LEE, Hongcheol, Seoul 06772 (KR); KANG, Eunseck, Seoul 06772 (KR); JUNG, Kyungho, Seoul 06772 (KR); CHO, Sanggeun, Seoul 06772 (KR); LEE, Juchul, Seoul 06772 (KR); LEE, Misun, Seoul 06772 (KR); LIM, Eunja, Seoul 06772 (KR)
(74) Representative: Vossius & Partner Patentanwälte Rechtsanwälte mbB
(86) International application number: PCT/KR2020/010027
(87) International publication number: WO 2022/025317

(57) **Abstract**

The method for producing a nanocellulose support comprises coating a container with surface-treated nanocellulose solution, forming a nanocellulose thin film by drying the coated nanocellulose solution, and modifying the surface properties of the nanocellulose thin film by means of electron beam irradiation. According to an embodiment, the production of nanocellulose supports using the drying method allows substrates of various shapes to be coated and has simple processes, thus allowing mass production and production of over-sized supports.

## Description

### TECHNICAL FIELD

Embodiments relate to a nanocellulose support and a method for producing the same.

### BACKGROUND ART

Cell therapy is a direct and groundbreaking method of treating diseases by injecting living cells into an affected area and has recently gained the most interest in the bio industry. Due to the nature of the treatment method in which cells have to be directly injected into the patient, one of the most important considerations when developing the treatments is the quality of the injected cells, that is, the safety of heteroimmunity and mutation of the cells.

Nanocellulose is a crystalline portion of cellulose, which is the main component of plant cell walls, and is an eco-friendly material with excellent mechanical properties due to hydrogen bonding between molecules. Due to the nature of the material extracted from plants, the nanocellulose is a non-animal and biocompatible material, and thus, there are fewer issues of heteroimmunity or cell mutation compared to the existing animal-derived cell culture materials. In addition, the nanocellulose is known to be advantageous for cell culture because the nanocellulose has a similar structure to the extracellular matrix (ECM). In spite of these advantages, since the nanocellulose is not easy to be attached to cells, it is difficult to mass-produce cells, and it is used only for research purposes as a type of culture in a hydrogel.

### DISCLOSURE OF THE INVENTION

### TECHNICAL PROBLEM

An object of embodiments is to solve the above and other problems.

Another object of embodiments is to provide a nanocellulose support suitable for cell culture and a method for producing the same.

Further another object of embodiments is to provide a nanocellulose support capable of mass-producing cells and a method for producing the same.

### TECHNICAL SOLUTION

According to an aspect of an embodiment to achieve the above or other object, a method for producing a nanocellulose support includes: applying a surface-treated nanocellulose solution to a container; drying the applied nanocellulose solution to form a nanocellulose thin film; and modifying surface properties of the nanocellulose thin film by using electron beam irradiation.

According to another aspect of an embodiment, a method for producing a nanocellulose support includes: dropping a plurality of beads into a container; dropping a surface-treated nanocellulose solution into the container; performing a drying process to form a nanocellulose thin film configured to surround an outer circumferential surface of each of the plurality of beads; and modifying surface properties of the nanocellulose thin film by using electron beam irradiation.

### ADVANTAGEOUS EFFECTS

The nanocellulose support according to the embodiments and the effects of the method for producing the same are described as follows.

According to at least one of the embodiments, there may be the advantage in that the wettability of the culture solution is improved by the hydrophilizing the nanocellulose thin film formed by the drying process through the electron beam irradiation.

According to at least one of the embodiments, there may be the advantage in that the production of the nanocellulose support using the drying method allows the substrates having various shapes to be coated and has simple processes, thus allowing the mass production and the production of the over-sized support.

According to at least one of the embodiments, there may be the advantage in that the wettability of the culture solution is improved through the hydrophilic treatment to remove the microbubbles in the fiber, thereby improving the cell attachment.

According to at least one of the embodiments, there may be the advantage in that the nanocellulose support is easily decomposed using the plant degradation enzyme to minimize the damage of the cells compared to the method of collecting the cells by cutting the attached site of the cell by using the existing animal degradation enzyme, and the nanocellulose support is decomposed in the short time compared to the existing hydrogel to obtain the cells having the excellent quality in the short time.

According to at least one of the embodiments, there may be the advantage in that it is possible to provide the cell culture method suitable for the mass production in the simple and fast process by overcoming the material limitations of the nanocellulose, thereby significantly contributing to the bio industries in the future.

The additional scope of the applicability of the embodiments will become apparent from the detailed description below. However, the various changes and modifications within the spirit and scope of the embodiments may be clearly understood by those skilled in the art, and thus, specific embodiments such as the detailed description and the preferred embodiments should be understood as given only as examples.

### BRIEF DESCRIPTION OF THE DRAWINGS

FIG. 1 is a flowchart for explaining a method for producing a nanocellulose support according to a first embodiment.
FIG. 2 is a view illustrating a process of applying a surface-treated nanocellulose solution to a container.
FIG. 3 is a view illustrating a process of forming a nanocellulose thin film by a drying process.
FIG. 4 is a view illustrating a process of modifying surface properties of the nanocellulose thin film by electron beam irradiation.
FIG. 5 is a view illustrating a change of a hydroxyl group according to each process.
FIG. 6 is a view illustrating a state in which air bubbles are collected in the nanocellulose thin film.
FIG. 7 is a view illustrating a state in which cells are not attached during cell culture due to air bubbles collected in the nanocellulose thin film.
FIG. 8 is a view illustrating a state in which air bubbles of the nanocellulose thin film are removed.
FIG. 9 is a view illustrating a state in which the cells are easily attached during the cell culture by removing the air bubbles of the nanocellulose thin film.
FIG. 10a is a view illustrating a state in which the cells are attached in the nanocellulose thin film formed on the basis of cationic nanocellulose.

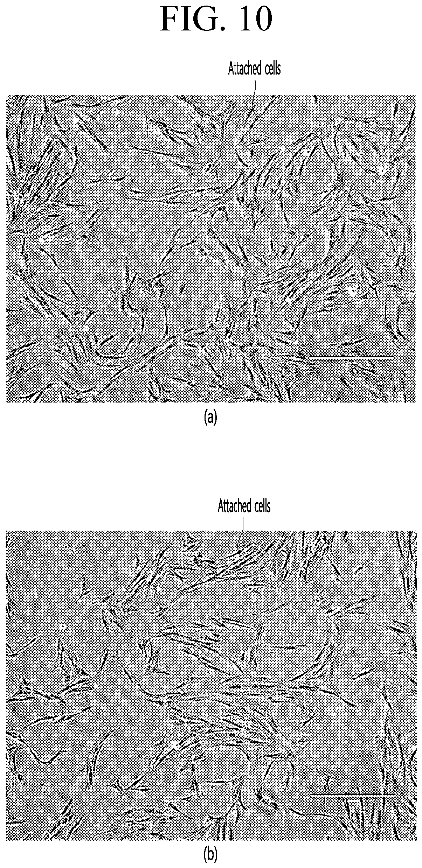

FIG. 10a is a view illustrating a state in which the cells are attached in the nanocellulose thin film formed on the basis of anionic nanocellulose.

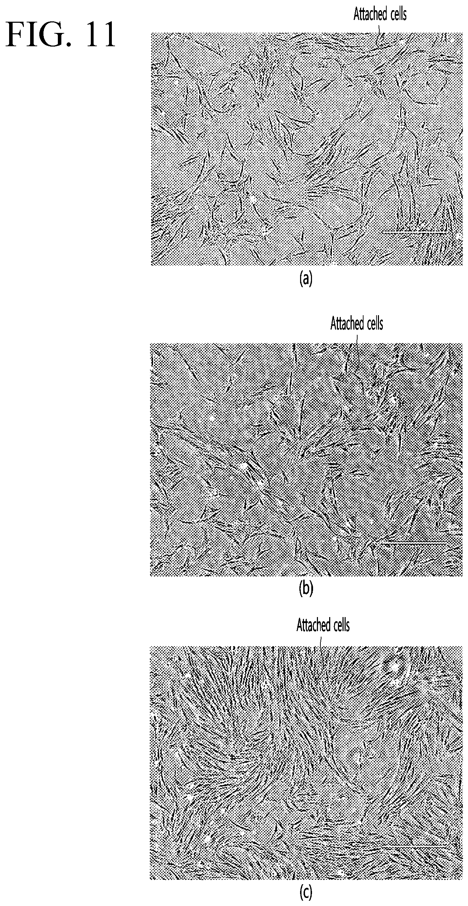

FIG. 11 is a view illustrating a state in which the cells are attached according to a flow rate in an O₂ plasma irradiation process.

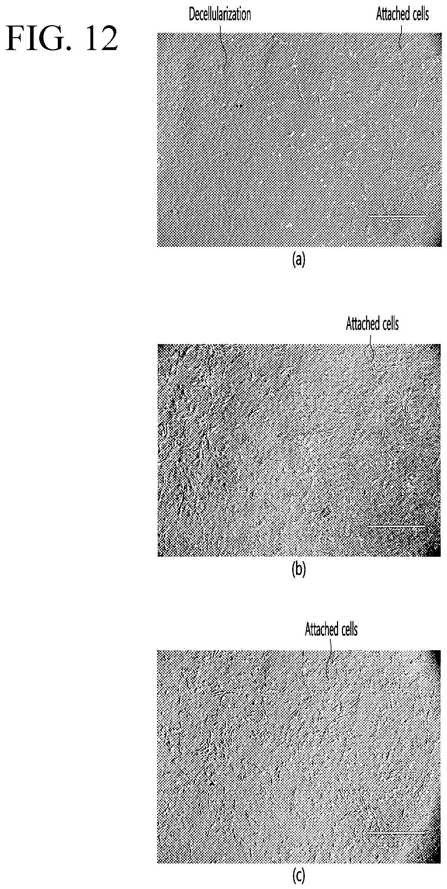

FIG. 12 is a view illustrating a state in which the cells are attached according to power in an O₂ plasma irradiation process.

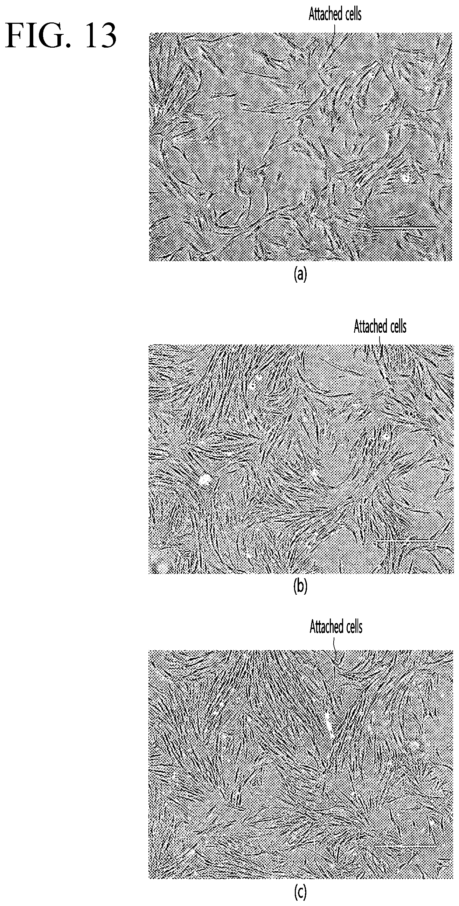

FIG. 13 is a view illustrating a state in which the cells are attached over time in an O₂ plasma irradiation process.

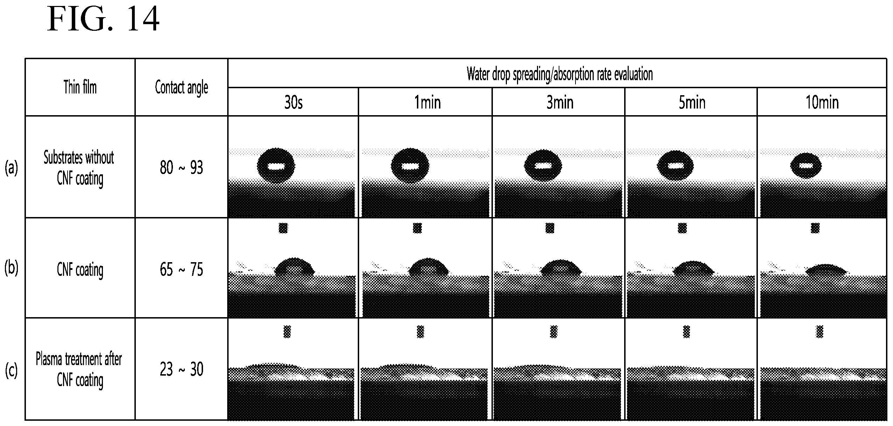

FIG. 14 is a view illustrating hydrophilicity evaluation results.

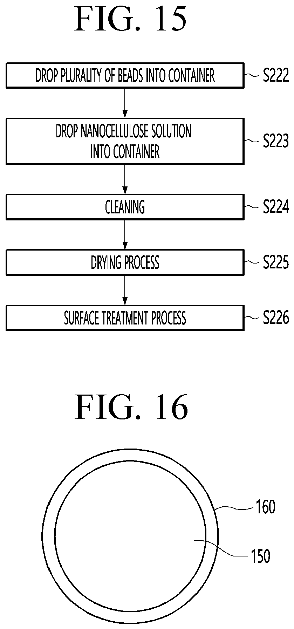

FIG. 15 is a flowchart for explaining a method for producing a nanocellulose support according to a second embodiment.
FIG. 16 is a view illustrating a portion of the nanocellulose support according to the second embodiment.

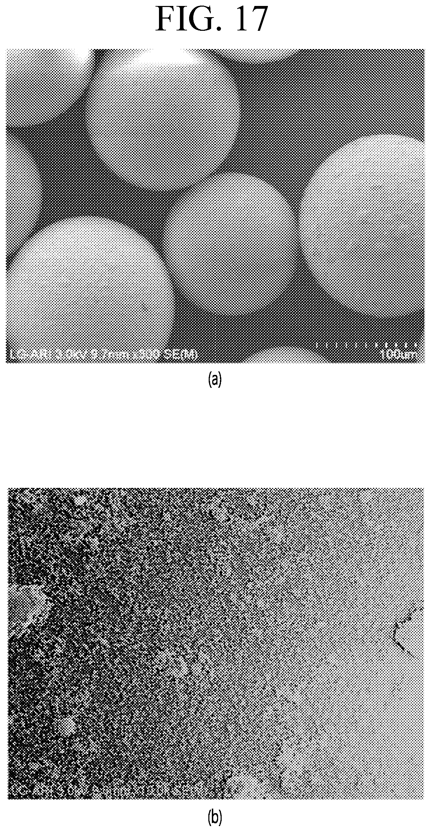

FIG. 17 is a view illustrating a pure bead.

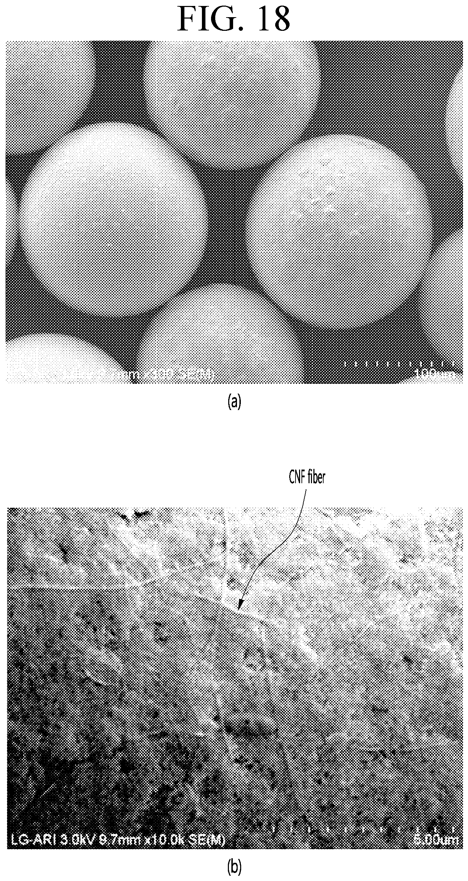

FIG. 18 is a view illustrating a nanocellulose thin film applied on the bead when containing 0.03% by weight of nanocellulose.

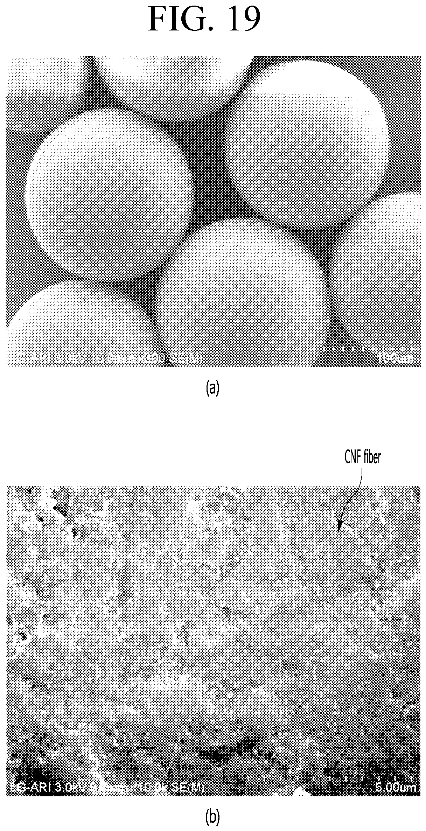

FIG. 19 is a view illustrating a nanocellulose thin film applied on the bead when containing 0.06% by weight of nanocellulose.

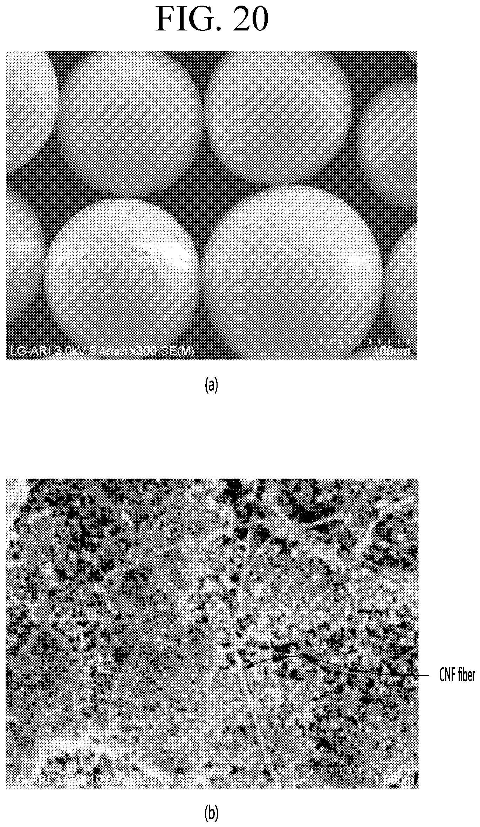

FIG. 20 is a view illustrating a nanocellulose thin film applied on the bead when containing 0.12% by weight of nanocellulose.

### MODE FOR CARRYING OUT THE INVENTION

Hereinafter, preferred embodiments of the present invention will be described below in more detail with reference to the accompanying drawings. However, the technical spirit of the present invention is not limited to some embodiments described, but may be implemented in various different forms, and within the technical spirit scope of the present invention, one or more of the components between the embodiments may be selectively coupled and substituted for the use. In addition, terms (including technical and scientific terms) used in the embodiments of the present invention, unless explicitly defined and described, can be generally understood by those skilled in the art to which the present invention pertains, and meanings of the terms, which are commonly used, such as predefined terms may be interpreted by considering the contextual meaning of the related technology. In addition, the terms used in the embodiments of the present invention are used only for explaining a specific exemplary embodiment while not limiting the present invention. In the present specification, a singular form may also include a plural form unless specifically stated in the phrase, and when described as "at least one (or more than one) of B, and (or) C", a combination of A, B, and C can contain one or more of all possible combinations. In the description of the components of the present invention, the terms first, second, A, B, (a), and (b) may be used. Each of the terms is merely used to distinguish the corresponding component from other components, and does not delimit an essence, an order or a sequence of the corresponding component. In addition, when any component is described as being 'connected', 'coupled' or 'linked' to another component, not only the component is directly connected, coupled, or linked to the other component, but also to the component is 'connected', 'coupled' or 'linked' by another component between the other components. In addition, when described as being formed or disposed in the "upper (top) or below (bottom)" of each component, the upper (top) or below (bottom) is not only when the two components are in direct contact with each other, but also a case in which another component described above is formed or disposed between the two components. In addition, when expressed as "upper (top) or below (bottom)", it may include the meaning of the downward direction as well as the upward direction based on one component.

### [First Embodiment]

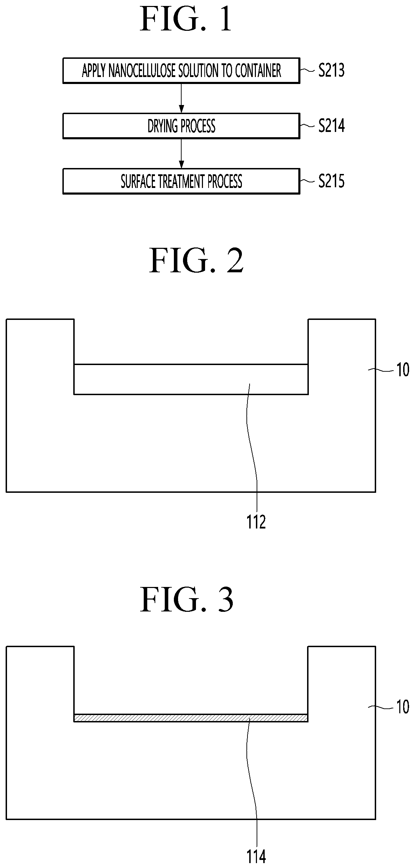

FIG. 1 is a flowchart for explaining a method for producing a nanocellulose support according to a first embodiment.

Referring to FIG. 1, a surface-treated nanocellulose solution may be applied to a container (S213).

For example, the nanocellulose solution may be produced by dispersing nanocellulose into a solution.

The nanocellulose is a crystalline portion of cellulose, which is the main component of a plant cell wall, and may be composed of nano-sized particles. For example, the nanocellulose may be produced by crushing raw wood and performing a series of processing procedures. In these processing procedures, a procedure for allowing the nanocellulose to have hydrophilicity may be included.

The nanocellulose may be, for example, nanocellulose surface-treated with anions. The anion may include, for example, a reactive group such as a sulfonic acid group, a phosphonic acid group, a carboxyl group, a sulfuric acid group, or a phosphoric acid group.

The nanocellulose may be, for example, nanocellulose surface-treated with cations. The cation may include, for example, an amine group including an epoxypropyltrimethylammonium group, a diethylaminoethyl group, and a dimethylamionethyl group, an amide group, an amino group, an ammonium group, a phosphonium group, and a sulfonium group.

FIG. 10a is a view illustrating a state in which cells are attached in the nanocellulose thin film formed on the basis of cationic nanocellulose, and FIG. 10a is a view illustrating a state in which the cells are attached in the nanocellulose thin film formed on the basis of anionic nanocellulose. The nanocellulose thin film may be a cellulose nano fiber (CNF).

It is seen that an amount of cell attachment in the nanocellulose thin film formed on the basis of cationic nanocellulose (FIG. 10a) and an amount of cell attachment in the nanocellulose thin film formed on the basis of anionic nanocellulose (FIG. 10b) are almost similar to each other, and also both the amounts of cell attachment are good.

Therefore, when the nanocellulose thin film is formed based on the cationic nanocellulose or the anionic nanocellulose according to the first embodiment, mass production of the cells may be possible.

A concentration of the nanocellulose may be 0.01% to 5% by weight. Other concentrations other than these concentrations of nanocellulose may be solutions. For example, the concentration of the nanocellulose may be 0.01% to 2% by weight. For example, the concentration of the nanocellulose may be 0.03% to 1% by weight.

The solution may be, for example, distilled water. The solution may be, for example, sterilized bio-distilled water. The solution may be, for example, a mixed solution in which one of distilled water or sterilized bio-distilled water and ethanol are mixed.

Since the nanocellulose is treated to be hydrophilic, the nanocellulose dispersed in the nanocellulose solution may also be maintained with hydrophilicity. Thus, the nanocellulose solution may be called a surface-treated nanocellulose solution.

For example, the surface-treated nanocellulose solution may be applied to a container.

As illustrated in FIG. 2, a container 10 may be provided. The container 10 may have a shape having an empty space therein or may have a flat shape. For example, as will be described later, the container 10 may be used as a cell culture dish by directly forming the nanocellulose thin film therein.

The surface-treated nanocellulose solution 112 may be applied to a bottom surface of the container 10. The applied nanocellulose solution may be dried to form the nanocellulose thin film (S214).

For example, the drying process may be performed by a heat source such as a heater or an oven.

A drying temperature may be, for example, 50 degrees to 100 degrees. A drying temperature may be, for example, 60 degrees to 90 degrees.

As illustrated in FIG. 3, the nanocellulose solution 112 in the container 10 may be evaporated by the drying process, and thus, the nanocellulose thin film 114 may be formed.

Thus, a nanocellulose support in which the nanocellulose thin film 114 is formed directly on the container 10 may be produced.

The nanocellulose support having modified surface properties of the nanocellulose thin film may be produced using electron beam irradiation (S215).

The nanocellulose support may include the container 10 and the nanocellulose thin film 114 disposed in the container 10 to increase in hydrophilicity.

The electron beam irradiation may be, for example, O₂ plasma or UV/O₃ irradiation.

In the embodiment, the O₂ plasma is described for convenience of explanation, but the electron beam irradiation may be performed using other gas plasma.

For example, the O₂ plasma irradiation process may be performed with an O₂ flow rate of 0.1 sccm to 150 sccm, power of 30 W to 200 W, and a time of 5 seconds to 300 seconds.

FIG. 11 is a view illustrating a state in which the cells are attached according to a flow rate in the O₂ plasma irradiation process.

FIG. 11 illustrates cell culture experimental results using the nanocellulose support including the nanocellulose thin film according to an embodiment.

It is seen that the cell culture is good at O₂ flow rates of 70 sccm (FIG. 11a), 50 sccm (FIG. 11b), and 30 sccm (FIG. 11c). Particularly, it is seen that the lower the O₂ flow rate, the higher the amount of cell attachment.

Thus, the O₂ flow rate may be 0.5 sccm to 100 sccm. For example, the O₂ flow rate may be 20 sccm to 50 sccm.

FIG. 12 is a view illustrating a state in which the cells are attached according to power in an O₂ plasma irradiation process.

FIG. 12 illustrates cell culture experimental results using the nanocellulose support including the nanocellulose thin film according to an embodiment.

Although the cell culture is performed at power of 50 W (FIG. 12a), decellularization of some of the cells occurred.

It is seen that the amount of cell attachment significantly increases in the power of 100 W (FIG. 12b) or 200 W (FIG. 12c).

Thus, the power may be 50 W to 200 W. For example, the power may be 80 W to 200 W. For example, the power may be 100 W to 200 W.

FIG. 13 is a view illustrating a state in which the cells are attached over time in an O₂ plasma irradiation process.

FIG. 13 illustrates cell culture experimental results using the nanocellulose support including the nanocellulose thin film according to an embodiment.

It is seen that the cell culture is good when an O₂ plasma irradiation time is 20 seconds (FIG. 13a), 40 seconds (FIG. 13b), and 60 seconds (FIG. 13b). Particularly, it is seen that the amount of cell attachment increases as the O₂ plasma irradiation time increases.

Thus, the O₂ plasma irradiation time may be 10 seconds to 200 seconds. For example, the O₂ plasma irradiation time may be 15 seconds to 100 seconds. For example, the O₂ plasma irradiation time may be 20 seconds to 70 seconds.

For example, in an UV/O₃ irradiation process, an electron beam is irradiated with UV having a wavelength of 160 nanometers to 300 nanometers for 1 minute to 4 hours, and thus, the hydrophilicity of the surface of the nanocellulose thin film may increase.

For example, in an UV/O₃ irradiation process, an electron beam is irradiated with UV having a wavelength of 180 nanometers to 260 nanometers for 3 minute to 2 hours, and thus, the hydrophilicity of the surface of the nanocellulose thin film may increase.

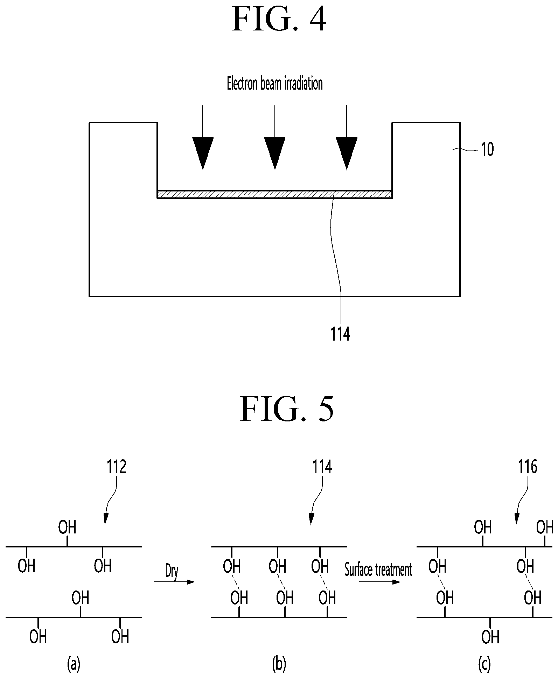

As illustrated in FIG. 4, the electron beam may be irradiated toward the inside of the container 10.

The surface of the nanocellulose thin film 116 may be modified by the electron beam. For example, the hydrophilicity of the surface of the nanocellulose thin film 116 may increase by the electron beam.

For example, a hydroxyl group (-OH) closed by a hydrogen bond through the drying process may be reactivated by the electron beam irradiation.

As illustrated in FIG. 5a, the nanocellulose dispersed in the nanocellulose solution 112 may have hydrophilicity including the hydroxyl group (-OH).

However, as illustrated in FIG. 5b, the hydroxyl group (-OH) may be closed by the hydrogen bond in the drying process (S214), and thus, the hydroxyl group (-OH) may be hidden inside the nanocellulose thin film so as not to be exposed to the outside of the nanocellulose thin film, thereby reducing the hydrophilicity. Therefore, when the cell culture is performed on the nanocellulose thin film having the reduced hydrophilicity, mass production of the cells may be difficult because the cells are not well attached to the nanocellulose thin film.

Therefore, as illustrated in FIG. 5c, in the first embodiment, the electron beam irradiation process, that is, the surface treatment process may be performed (S215), and the electron beam may be irradiated to the nanocellulose thin film 116 to reactivate the hydroxyl group (-OH). As a result, the hydroxyl group (-OH) may be exposed to the outside of the nanocellulose thin film 116, and thus, the hydrophilicity of the nanocellulose thin film 116 may increase. Therefore, when the cell culture is performed on the nanocellulose thin film 116 having the increased hydrophilicity, the cell attachment to the nanocellulose thin film 116 may be facilitated, and thus, the mass production of the cells may be possible.

As another example, air bubbles collected inside the nanocellulose thin film 116 by the drying process may be removed due to the increased hydrophilicity by the electron beam irradiation.

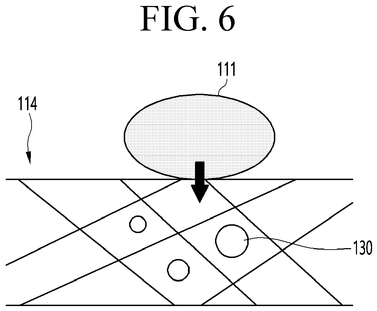

As illustrated in FIG. 6, when the drying process is performed (S214), the nanocellulose solution 112 may be evaporated by the drying process, and in this case, the hydrophilicity of the surface of the nanocellulose thin film may be reduced. Thus, air in the nanocellulose thin film may not leak to the outside, and thus, air bubbles 130 may be collected in the nanocellulose thin film. Since each of the air bubbles 130 have a fine size, the air bubbles may be called microbubbles.

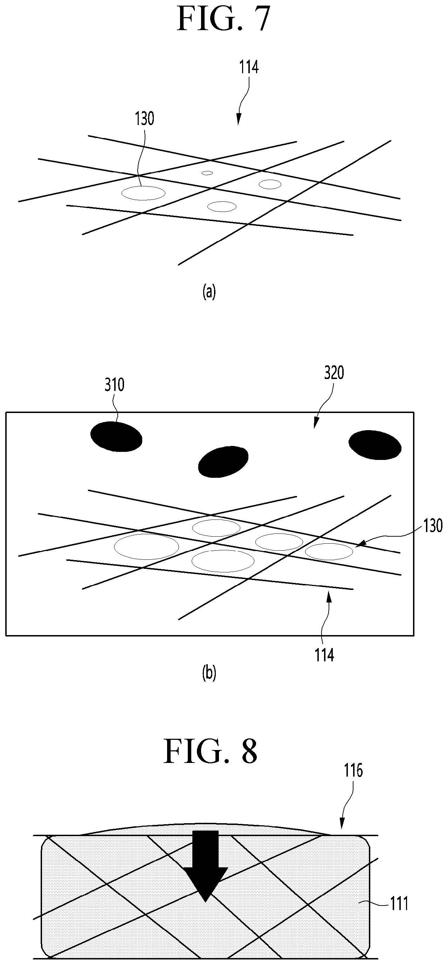

As illustrated in FIG. 7a, the air bubbles 130 may be collected in the nanocellulose thin film. In the case of the cell culture in the nanocellulose thin film in which the air bubbles 130 are collected, as illustrated in FIG. 7b, the cells 310 included in the culture solution 320 may not be well attached to the nanocellulose thin film due to the air bubbles 130 collected in the nanocellulose thin film, and thus, the mass production of the cells 310 may be difficult.

On the other hand, as in the first embodiment, when the surface treatment process is performed by the electron beam irradiation (S215), the hydrophilicity of the surface of the nanocellulose thin film 116 may increase. Thus, while the nanocellulose solution penetrates into the nanocellulose thin film 116, the bubbles 130 in the nanocellulose thin film 116 are pushed out to remove the air bubbles 130 inside the nanocellulose thin film 116.

As illustrated in FIG. 8, when the surface treatment process is performed by the electron beam irradiation (S215), the hydrophilicity of the surface of the nanocellulose thin film 116 may increase by the electron beam irradiation process, and thus, the nanocellulose solution may be easily penetrated into the nanocellulose thin film 116. Thus, the air bubbles 130 may be pushed outward by the nanocellulose solution penetrated into the nanocellulose thin film 116, and thus, the air bubbles 130 may be removed from the inside of the nanocellulose thin film 116.

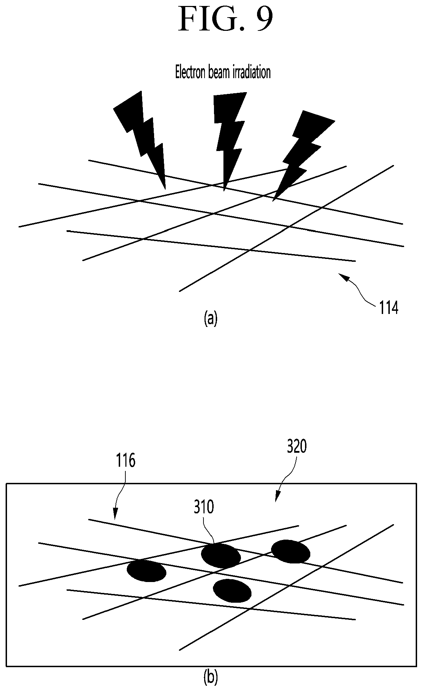

As illustrated in FIG. 9a, the air bubbles 130 in the nanocellulose thin film 116 may be removed due to the increase in hydrophilicity by the electron beam irradiation. In this case, as illustrated in FIG. 9b, the air bubbles 130 in the nanocellulose thin film 116 may be removed, and thus, the cells 310 included in the culture solution 320 may be well attached to enable the mass production.

FIG. 14 is a view illustrating hydrophilicity evaluation results.

As illustrated in FIG. 14a, when the container 10 is not coated with the nanocellulose thin film, a contact angle between the container 10 and a water droplet is 80 degrees to 93 degrees.

As illustrated in FIG. 14b, in the case of the container 10 coated with the nanocellulose thin film by the drying process (S214 in FIG. 1), the contact angle between the nanocellulose thin film and the water droplet may be 65 degrees to 75 degrees.

As illustrated in FIG. 14c, in the case of the container 10 coated with the nanocellulose thin film by the electron beam process (S215 in FIG. 1), the contact angle between the nanocellulose thin film and the water droplet may be 23 degrees to 30 degrees.

Therefore, as in the first embodiment, the nanocellulose thin film may have very high hydrophilicity by the electron beam irradiation process (S215 in FIG. 1). Therefore, the nanocellulose support including the highly hydrophilic cellulose nanofibers may be mass-produced by cells.

The cellulose nano-film according to the first embodiment may be used as one of a water treatment agent, a heavy metal adsorbent, and a fine dust blocking filter.

According to the first embodiment, wettability of the culture solution may be improved by hydrophilizing the nanocellulose thin film formed by the drying process through the electron beam irradiation.

According to the first embodiment, the production of nanocellulose supports using the drying method may allow the container having various shapes to be applied and have simple processes, thus enabling the mass production and production of over-sized supports.

According to the first embodiment, the wettability of the culture solution may be improved through the hydrophilic treatment to remove the microbubbles in the fiber, thereby improving the cell attachment.

According to at least one of the embodiments, the nanocellulose support may be easily decomposed using the plant degradation enzyme to minimize the damage of the cells compared to the method of collecting the cells by cutting the attached site of the cell by using the conventional animal degradation enzyme, and the nanocellulose support may be decomposed in the short time compared to the conventional hydrogel to obtain the cells having the excellent quality in the short time.

According to at least one of the embodiments, it may be possible to provide the cell culture method suitable for the mass production in the simple and fast process by overcoming the material limitations of the nanocellulose, thereby significantly contributing to the bio industries in the future.

### [Second Embodiment]

FIG. 15 is a flowchart for explaining a method for producing a nanocellulose support according to a second embodiment.

Referring to FIG. 15, a plurality of beads may be dropped into a container (S222). As illustrated in FIG. 16, the plurality of beads 150 may be circular particles. For example, each of the beads 150 may be made of a polymer material. For example, the bead 150 may include one of polystyrene-based material, polyolefin-based material, polyvinyl-based material, and polyethylene terephthalate.

The nanocellulose solution may be dropped into the container (S223).

For example, the nanocellulose solution may be produced by dispersing nanocellulose into a solution.

The nanocellulose may be, for example, nanocellulose surface-treated with anions. The anion may include, for example, a reactive group such as a sulfonic acid group, a phosphonic acid group, a carboxyl group, a sulfuric acid group, or a phosphoric acid group.

The nanocellulose may be, for example, nanocellulose surface-treated with cations. The cation may include, for example, an amine group including an epoxypropyltrimethylammonium group, a diethylaminoethyl group, and a dimethylamionethyl group, an amide group, an amino group, an ammonium group, a phosphonium group, and a sulfonium group.

Therefore, when the nanocellulose thin film is formed based on the cationic nanocellulose or the anionic nanocellulose according to the second embodiment, mass production of the cells may be possible.

A concentration of the nanocellulose may be 0.01% to 5% by weight. Concentrations other than the concentration of the nanocellulose may be concentrations of the solution. For example, the concentration of the nanocellulose may be 0.01% to 2% by weight. For example, the concentration of the nanocellulose may be 0.03% to 1% by weight.

The solution may be, for example, distilled water. The solution may be, for example, sterilized bio-distilled water. The solution may be, for example, a mixed solution in which one of distilled water or sterilized bio-distilled water and ethanol are mixed.

Since the nanocellulose is treated to be hydrophilic, the nanocellulose dispersed in the nanocellulose solution may also be maintained with hydrophilicity. Thus, the nanocellulose solution may be called a surface-treated nanocellulose solution.

For example, a nanocellulose solution (112 in FIG. 2) may be dropped into a container (10 in FIG. 2) into which a plurality of beads 150 are dropped. As another example, the nanocellulose solution (112 in FIG. 2) may be dropped first into the container and then the plurality of beads 150 may be dropped. As further another example, the nanocellulose solution (112 in FIG. 2) and a plurality of beads may be dropped at the same time. An amount of nanocellulose solution (112 in FIG. 2) may be determined according to a diameter of the bead 150. That is, the nanocellulose solution may be dropped higher than the highest point of the plurality of beads 150, and the plurality of beads 150 may be immersed in the nanocellulose solution (112 in FIG. 2) .

Therefore, a minimum amount of nanocellulose solution (112 in FIG. 2), at which the plurality of beads 150 are immersed, may be dropped into the container (10 in FIG. 2) to minimize consumption of the nanocellulose solution (112 in FIG. 2), thereby saving costs.

Although not shown, after the nanocellulose solution (112 in FIG. 2) and the plurality of beads 150 are dropped into the container (10 in FIG. 2), the container 10 may rotate to allow the plurality of beads 150 to flow within the nanocellulose solution (112 in FIG. 2) without sinking to a bottom surface of the container 10. As described above, as the plurality of beads 150 flow in the nanocellulose solution 112, the nanocellulose of the nanocellulose solution may be attached to an outer circumferential surface of the beads 150.

Thereafter, washing may be performed on the plurality of beads (S224).

For example, the nanocellulose solution 112 in the container (10 in FIG. 2) may be dropped onto a sieve (not shown), and the plurality of beads 150 in the nanocellulose solution 112 may be sieved through the sieve. Thus, the plurality of beads 150 remain on the sieve. Here, an outer circumferential surface of each of the plurality of beads 150 may be coated with the nanocellulose to a certain thickness.

Then, although not shown, sterilized water may be dropped onto the sieve to wash the plurality of beads 150 remaining on the sieve.

Thereafter, a drying process is performed (S225), and a nanocellulose thin film may be formed on the plurality of beads.

Thereafter, an electron beam irradiation process, that is, a surface treatment process (S226) may be performed to form the nanocellulose thin film having the increased hydrophilicity.

As illustrated in FIG. 16, the nanocellulose thin film 160 having the increased hydrophilicity may be formed on the surface of each of the beads 150 to produce a nanocellulose support.

The nanocellulose support may include a plurality of nanocellulose thin films 160 each of which has a bead shape and which have the increased hydrophilicity.

Various thin films each of which has the bead shape will be described with reference to FIGS. 17 to 20.

FIGS. 17a, 18a, 19a, and 20a are enlarged views illustrating the plurality of beads. FIGS. 17b, 18b, 19b, and 20b are views illustrating the plurality of beads.

FIGS. 17a and 17b illustrate pure beads, which are not coated with the nanocellulose thin film according to the embodiment.

FIGS. 18a and 18b illustrate a state a concentration of the nanocellulose is 0.03% by weight, FIGS. 19a and 19b illustrate a state when a concentration of the nanocellulose is 0.06% by weight, and FIGS. 20a and 20b illustrate a state when a concentration of the nanocellulose is 0.12% by weight.

As illustrated in FIGS. 18a to 20, it is seen that as the concentration of the nanocellulose increases, a cellulose nano film containing more cellulose nano fibers per unit area is produced.

The nanocellulose support including the nanocellulose thin film produced according to the second embodiment may be used as microcarriers.

The microcarrier may be a cell culture support for mass-cultivating cells. Thus, the cells may be cultured by filling a culture medium in a cell cultivator (or bioreactor), and suspending microcarriers to which the cells are attached in the culture medium.

Conventionally, a 2D cell cultivator has a small number of culturable cells per unit area, and it is possible to cultivate the cells through many manual processes by the skilled expert. On the other hand, if a 3D microcarrier is used, as in the example, the number of culturable cells per unit area may be large, and automation may be possible, and thus, many cells may be more easily mass-cultivated.

In addition, the nanocellulose support produced according to the second embodiment may be used as a bead for collecting biomaterials. Since modification of surface properties of the nanocellulose thin film acts as a functional group capable of antigen-antibody reaction of a target material, the nanocellulose support according to the second embodiment, that is, a plurality of spherical beads may be suspended in a culture solution containing the cells in the culture solution to collect the target material. Thereafter, the plurality of spherical beads sunk in the culture solution may be collected, and the nanocellulose thin film may be dissolved with a plant degrading enzyme to easily collect the desired target material. For example, cellulase, xylanase, pectinase, hemicellulase, sucrase, amylase, or a combination thereof may be used as the plant degrading enzyme.

Therefore, according to the second embodiment, the nanocellulose thin film may be melted with the plant degrading enzyme to minimize damage of the cells compared to the conventional method of collecting the cells by cutting attachment sites of the cells with an animal degrading enzyme.

According to the second embodiment, it is possible to decompose in a short time compared to the conventional hydrogel, and thus, the cells having excellent quality may be obtained in a short time.

The nanocellulose support produced according to the first and/or second embodiments may be applied as a water treatment bead for removing heavy metals if the functional group capable of removing the heavy metals is attached and may also be used as a fine dust blocking filter.

The nanocellulose is known to have heavy metal and contaminant adsorption properties. Therefore, when developing the nanocellulose filter using the nanocellulose support produced according to the first and/or second embodiment, the wettability to water may be improved, and the entire area of the filter may be activated in a short time to maximize the filter characteristics. Therefore, it is expected that the contribution to the environmental industry will be high.

The detailed description is intended to be illustrative, but not limiting in all aspects. It is intended that the scope according to the embodiment should be determined by the rational interpretation of the claims as set forth, and the modifications and variations according to the embodiment come within the scope of the appended claims and their equivalents.

### INDUSTRIAL APPLICABILITY

The embodiment may be applied to various industries such as the bio industry and the environmental pollution industry.

## Claims

1. A method for producing a nanocellulose support, the method comprising:
applying a surface-treated nanocellulose solution to a container;
drying the applied nanocellulose solution to form a nanocellulose thin film; and
modifying surface properties of the nanocellulose thin film by using electron beam irradiation.

2. The method of claim 1, wherein the container has a shape with an empty space therein or a planar shape.

3. The method of claim 1, wherein the nanocellulose is surface-treated with at least one of cations and anions.

4. The method of claim 1, wherein the hydrophilicity of the nanocellulose thin film is increased by the electron beam irradiation.

5. The method of claim 1, wherein the nanocellulose solution contains distilled water or a mixed solution of the distilled water and ethanol and 0.01% to 5% by weight of nanocellulose.

6. The method of claim 1, wherein the electron beam irradiation comprises irradiation by one of gas plasma and UV/O₃.

7. The method of claim 6, wherein the gas plasma irradiation process is performed at an O₂ flow rate of 0.1 sccm to 150 sccm and power of 50 W to 200 W for a time of 5 seconds to 300 seconds.

8. A nanocellulose support produced by the method according to claim 1.

9. The nanocellulose support according to claim 8, wherein the nanocellulose support is used as one of a microcarrier, a water treatment agent, a heavy metal adsorbent, and a fine dust blocking filter.

10. A method for producing a nanocellulose support, the method comprising:
dropping a plurality of beads into a container;
dropping a surface-treated nanocellulose solution into the container;
performing a drying process to form a nanocellulose thin film configured to surround an outer circumferential surface of each of the plurality of beads; and
modifying surface properties of the nanocellulose thin film by using electron beam irradiation.

11. The method of claim 10, wherein the bead comprises a polymer bead containing one of polystyrene-based, polyolefin-based, polyvinyl-based, and polyethylene terephthalate.

12. The method of claim 10, wherein the nanocellulose is surface-treated with at least one of cations and anions.

13. The method of claim 10, wherein the hydrophilicity of the nanocellulose thin film is increased by the electron beam irradiation.

14. The method of claim 10, wherein the nanocellulose solution contains distilled water or a mixed solution of the distilled water and ethanol and 0.01% to 5% by weight of nanocellulose.

15. The method of claim 10, wherein the electron beam irradiation process is performed at an O₂ flow rate of 0.1 sccm to 150 sccm and power of 30W to 200 W for a time of 5 seconds to 300 seconds.

16. The method of claim 10, wherein the electron beam irradiation comprises irradiation by one of gas plasma and UV/O₃.

17. A nanocellulose support produced by the method according to claim 10.

18. The nanocellulose support according to claim 17, wherein the nanocellulose support is used as one of a microcarrier, a water treatment agent, a heavy metal adsorbent, and a fine dust blocking filter.
